# FASCICULE DE BREVET EUROPEEN

(11) **EP 1 433 497 B1**
(45) Date de publication et mention de la délivrance du brevet: **03.05.2006**
(21) Numéro de dépôt: 03293303.8
(22) Date de dépôt: 23.12.2003
(51) Int. Cl.: A61N 1/37

(54) **Dispositif médical implantable actif à détection des potentiels cardiaques évoqués post-stimulation, notamment auriculaires**
Aktive implantierbare medizinische Vorrichtung mit Detektierung von evozierten Herzpotentialen, insbesondere Atrialpotentialen
Active implantable medical device with detection of cardiac, in particular atrial, evoked potentials

(30) Priorité: 26.12.2002 FR 0216724
(43) Date de publication de la demande: 30.06.2004
(73) Titulaire: ELA MEDICAL, 92541 Montrouge (FR)
(72) Inventeur: Legay, Thierry, 91640 Fontenay les Bris (FR); DeCoene, Dominique, 78760 Jouars Pontchartrain (FR)
(74) Mandataire: Dupuis-Latour, Dominique

(56) Documents cités:
- EP-A- 0 962 235
- WO-A-01/43819
- US-A- 4 674 509
- US-A- 4 825 870

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Tous ces dispositifs comportent des moyens de détection d'activité, c'est-à-dire de détection des dépolarisations spontanées du myocarde, ainsi que des moyens de stimulation de ce myocarde.

Après la stimulation, il est important de pouvoir recueillir I"'onde évoquée", c'est-à-dire l'onde de dépolarisation induite par la stimulation de la cavité considérée, afin de déterminer si cette stimulation a été efficace ou non, par exemple pour ajuster l'amplitude et/ou la largeur de l'impulsion de stimulation.

La difficulté tient au fait que l'onde évoquée est très précoce et qu'en sortie de l'amplificateur de détection elle se trouve mélangée, voire noyée, dans le transitoire électrique appelé "réponse de l'amplificateur" consécutif à la stimulation électrique. Cette réponse est toujours présente indépendamment de la présence d'une onde évoquée ou non, et ne dépend que des caractéristiques électriques de l'amplificateur lui-même, de l'énergie contenue dans l'impulsion de stimulation (amplitude et largeur) ainsi que des caractéristiques d'impédance de l'interface coeur/électrode, appelées "polarisation de la sonde".

En particulier, pour écouler les charges à l'interface coeur/électrode après la stimulation, on prévoit d'effectuer une décharge de l'énergie accumulée, car sinon les stimulations suivantes finiraient par ne plus être efficaces. Pendant cette phase de décharge, il est prévu une déconnexion ou "blanking" des circuits de détection, typiquement pendant une durée de l'ordre de 14 ms, la stimulation ayant elle-même une durée de l'ordre de 1 ms.

De plus, au moment de la reconnexion de l'amplificateur aux bornes de l'électrode de détection à l'issue de la période de blanking, apparaît en sortie de l'amplificateur un rebond transitoire de tension, qui perdure quelques millisecondes jusqu'à se que l'amplificateur soit complètement désaturé.

La recherche de l'onde évoquée est encore plus difficile dans le cas d'une stimulation atriale. En effet, l'onde évoquée présente alors une amplitude beaucoup plus faible que dans le cas du ventricule, et elle est en outre beaucoup plus précoce : ainsi, l'onde évoquée auriculaire (onde P) apparaît environ 10 ms après stimulation pour se terminer à environ 30 ms, alors que l'onde évoquée ventriculaire (onde R) est observée environ 60 ms après la stimulation.

On comprendra que, dans ces conditions, il soit très difficile de détecter la présence d'une onde P évoquée, par exemple dans le cas d'un test de capture atriale.

L'un des buts de l'invention est de remédier à cette difficulté, en proposant une nouvelle technique de détection de l'onde P évoquée tenant compte du caractère à la fois très précoce et de faible amplitude de cette dernière.

On notera cependant que l'invention n'est pas limitée au recueil de l'onde P évoquée, mais peut être appliquée aussi bien, compte tenu des avantages multiples qu'elle procure, au recueil des ondes R évoquées (ondes ventriculaires), par exemple dans le cas d'un test de capture ventriculaire.

Comment on le verra, la technique de détection de l'onde évoquée selon l'invention est capable de s'affranchir de la polarisation de la sonde quel que soit le niveau d'énergie utile de l'impulsion de stimulation, en permettant la détection d'une éventuelle onde P au cours d'une période, dite "période d'écoute", à la fois très précoce et prolongée, pouvant par exemple s'étendre typiquement de 6 ms à 40 ms après la stimulation dans le cas où la cavité stimulée est l'oreillette.

Plus précisément, le dispositif de l'invention est d'un type comprenant, de manière en elle-même connue : une borne apte à être reliée à une électrode intracardiaque d'une sonde implantée dans une première cavité cardiaque ; des moyens de stimulation, aptes à appliquer une impulsion de stimulation à la borne pour délivrance de cette impulsion à la première cavité ; des moyens de décharge couplés à la borne, aptes à court-circuiter cette borne pendant la durée d'une période de décharge ; et au moins un étage apte à détecter sur la borne des signaux électriques correspondant à des potentiels spontanés apparaissant dans la première cavité, et comportant un amplificateur avec une entrée couplée à la borne et une sortie, et des moyens de blanking comportant un commutateur apte à découpler temporairement la borne d'avec l'entrée de l'amplificateur. Le dispositif comprend en outre des moyens séquenceurs aptes à activer les moyens de décharge, après application de l'impulsion de stimulation, pendant la durée de la période de décharge, puis activer les moyens de blanking pendant au moins la durée de la stimulation et la période de décharge.

Un tel dispositif est connu du document WO-A-01/43819.

Selon l'invention, les moyens séquenceurs, après avoir activé les moyens de blanking une première fois pendant la durée de l'impulsion de stimulation, sont aptes à : activer les moyens de décharge avec délai après l'application de l'impulsion de stimulation ; puis inhiber les moyens de blanking pendant une période d'écoute incluant au moins une partie dudit délai, de manière à autoriser la détection par l'amplificateur, pendant cette période d'écoute, des signaux correspondant à des potentiels spontanés post-stimulation dans la première cavité ; et enfin activer les moyens de blanking une seconde fois après la période d'écoute, pendant la durée de la période de décharge.

Avantageusement, l'amplificateur est un amplificateur commutable entre un mode de détection normale et un mode de désaturation rapide, notamment un mode à fréquence de coupure basse et un mode à fréquence de coupure élevée, respectivement, et les moyens séquenceurs sont aptes, en outre, à commuter l'amplificateur en mode de désaturation rapide pendant au moins la durée de la période d'écoute. De préférence, les moyens séquenceurs recommutent l'amplificateur en mode de détection normale après le début de la période de décharge, et de façon non concomitante à ce début.

Très avantageusement, les moyens séquenceurs commandent les moyens de décharge de manière à mettre fin à la période de décharge avant la fin d'une période réfractaire post-stimulation d'analyse du signal en sortie de l'amplificateur, à la fois pour ladite première cavité et pour au moins une deuxième cavité cardiaque, de manière à éviter les fausses détections dans cette (ces) deuxième(s) cavité(s). Cette période réfractaire peut notamment être une période réfractaire redéclenchable de durée variable en fonction du niveau de perturbation présent en sortie de l'amplificateur.

Le dispositif peut comporter des moyens d'analyse du signal en sortie de l'amplificateur pendant la période d'écoute, aptes à détecter la présence d'une onde évoquée consécutive à la stimulation appliquée à la cavité, de manière à discriminer entre stimulations efficaces et stimulations non efficaces. Ces moyens d'analyse peuvent notamment comporter des moyens aptes à analyser les variations d'une dérivée d'ordre n, avec n =1, de préférence n = 2, du signal en sortie de l'amplificateur, en particulier en détectant le franchissement d'un seuil par un extremum de cette dérivée.

On va maintenant décrire un exemple de mise en oeuvre de l'invention, en référence aux dessins annexés.
La figure 1 est une vue schématique des circuits de stimulation et de détection mis en oeuvre dans le cadre du dispositif de l'invention.
La figure 2 montre le potentiel présent sur l'électrode de stimulation, dans le cadre d'un dispositif connu.
La figure 3 est homologue de la figure 2, pour le dispositif de l'invention.
La figure 4 est une série de chronogrammes illustrant la séquence des différentes opérations exécutées au sein du dispositif conformément aux enseignements de l'invention.
Les figures 5A et 5B montrent la variation de potentiel en sortie de l'amplificateur de détection après numérisation du signal, respectivement pour une stimulation inefficace et pour une stimulation efficace.
La figure 6 montre un résultat clinique illustrant la manière dont il est possible de discriminer nettement entre stimulations efficaces et stimulations inefficaces par analyse de la dérivée seconde du signal numérisé

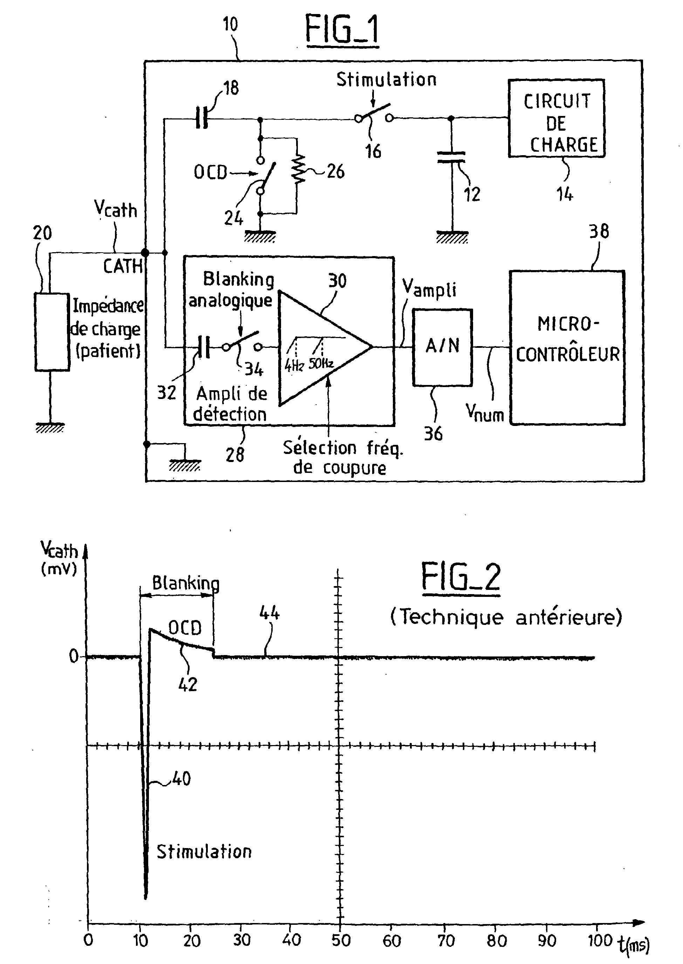

Sur la figure 1, la référence 10 désigne le circuit de l'invention, avec son étage de stimulation et son étage de détection.

L'étage de stimulation comporte un condensateur réservoir 12 chargé à une tension prédéterminée par un circuit de charge 14. L'énergie accumulée dans le condensateur 12 est délivrée pendant une durée prédéterminée (durée de l'impulsion de stimulation) par fermeture d'un interrupteur 16 assurant le transfert de cette énergie, via un condensateur de liaison 18, vers une borne de sortie CATH du stimulateur, borne reliée par exemple à l'électrode distale d'une sonde de stimulation auriculaire ou ventriculaire. Cette sonde et l'interface électrode/myocarde présentent ensemble une impédance schématisée en 20, constituant l'impédance de charge du dispositif (comprenant la résistance série de la sonde et du tissu cardiaque et la capacité de l'interface, dite "capacité de Helmholtz").

Le condensateur 18 a pour rôle de protéger le patient contre un éventuel courant continu en cas de défaillance des circuits de stimulation, par exemple du commutateur 16.

L'impulsion de stimulation présente typiquement une amplitude comprise entre 1,5 et 7,5 V avec une largeur d'impulsion comprise entre 0,12 ms et 0,98 ms (l'énergie de stimulation est proportionnelle à la largeur et au carré de l'amplitude de l'impulsion).

Après application d'une impulsion de stimulation, il est nécessaire de neutraliser les charges accumulées dans le condensateur 18 et l'impédance 20. Cette neutralisation est opérée par fermeture d'un interrupteur 24 réalisant électriquement la mise en série du condensateur de liaison 18 du dispositif et de l'impédance 20. Cette opération de décharge, souvent désignée "OCD" (*Output Capacitor Discharge*) doit être opérée à bref délai après la stimulation afin d'annuler le potentiel moyen dans l'interface myocarde/électrode car sinon, les stimulations suivantes seraient rapidement rendue inefficaces.

Une résistance 26, typiquement de 200 kO, permet d'achever la décharge du condensateur 18 après réouverture du commutateur 24. Elle permet également de définir l'impédance d'entrée globale de l'appareil pour la détection des signaux cardiaques.

En ce qui concerne l'étage de détection 28, celui-ci comporte un amplificateur-filtre 30 relié à la borne CATH via un condensateur de liaison 32 et un interrupteur commandé 34. Le signal Vₐₘₚₗᵢ en sortie de l'amplificateur 30 est échantillonné et numérisé par un convertisseur analogique/numérique 36 délivrant en sortie un signal numérisé Vₙᵤₘ apte à être analysé par le microcontrôleur 38 du dispositif.

Le rôle de l'interrupteur 34 est d'assurer un "blanking" analogique, c'est-à-dire une déconnexion de l'entrée de l'amplificateur 30 pendant la durée de la stimulation et de l'OCD, afin d'éviter une saturation trop importante. Les potentiels lors de la simulation peuvent en effet atteindre plusieurs volts, ceux au début de l'OCD étant de l'ordre de la centaine de millivolts.

Comme illustré figure 2, qui illustre la manière classique d'opérer le blanking, la période de blanking débute légèrement avant l'impulsion de stimulation 40 pour se terminer légèrement après la fin de l'OCD 42. Pour une durée d'OCD typique de 13 ms, la durée du blanking est de l'ordre de 14 ms afin que la période de blanking puisse complètement encadrer la stimulation et l'OCD suivant immédiatement cette stimulation.

Après la période d'OCD 42, le potentiel en entrée de l'amplificateur est à peu près stabilisé, mais en sortie l'amplificateur présente une réponse transitoire à la reconnexion post-blanking, excluant la détection d'une onde évoquée précoce.

Dans le cas d'une onde évoquée précoce, tout particulièrement d'une onde P (atriale) susceptible d'arriver aussi tôt que 10 ms après la stimulation, et avec de surcroît une faible amplitude et une courte durée (typiquement de 10 à 15 ms), l'onde évoquée peut être masquée pendant la période de blanking ou pendant la période d'instabilité post-blanking, décrite ci-dessus, s'étendant sur une quinzaine à une vingtaine de millisecondes après l'impulsion de stimulation. En effet, un transitoire important apparaît à la reconnexion post-blanking en sortie de l'amplificateur, qui rend très difficile la détection d'une onde évoquée atriale de faible amplitude, très précoce et très rapide.

Enfin, l'OCD peut être analysé comme un signal de pente relativement faible par rapport à l'impulsion de stimulation et d'amplitude très voisine de celle des complexes endocavitaires : dans cette hypothèse, si la décharge de l'interface n'est pas tout à fait terminée à la fin de l'OCD - ce qui est généralement le cas - la variation lente résiduelle présente sur le cathéter sera amplifiée de façon importante et créera un potentiel significatif au moment de la reconnexion post-blanking, empêchant de détecter facilement la présence d'un potentiel cardiaque évoqué superposé.

L'idée de base de la présente invention consiste à ne pas effectuer l'OCD immédiatement après la stimulation, mais à décaler cette phase de décharge après la période d'apparition de l'onde évoquée.

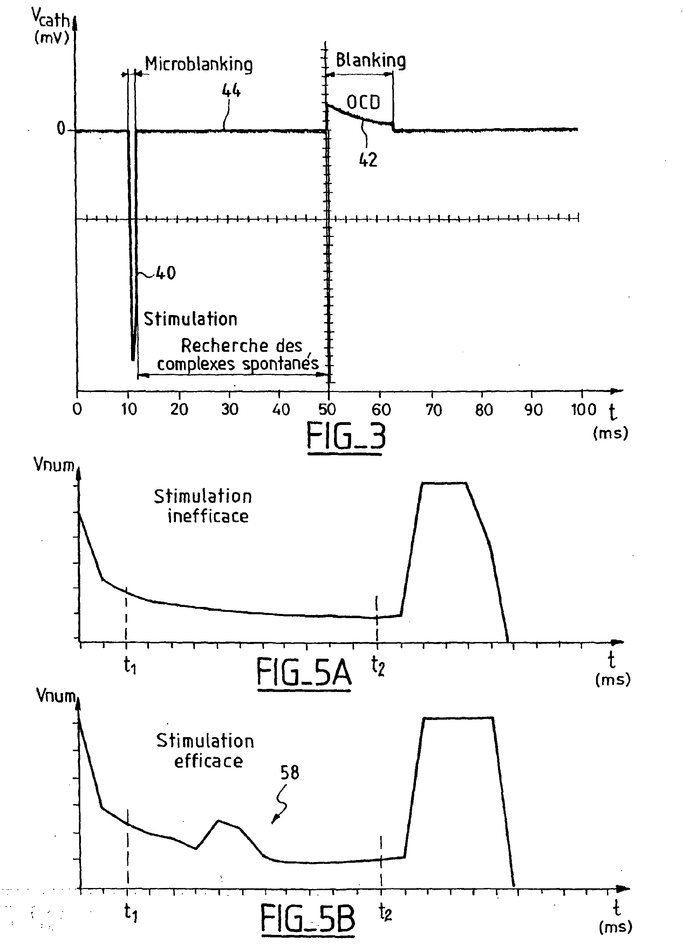

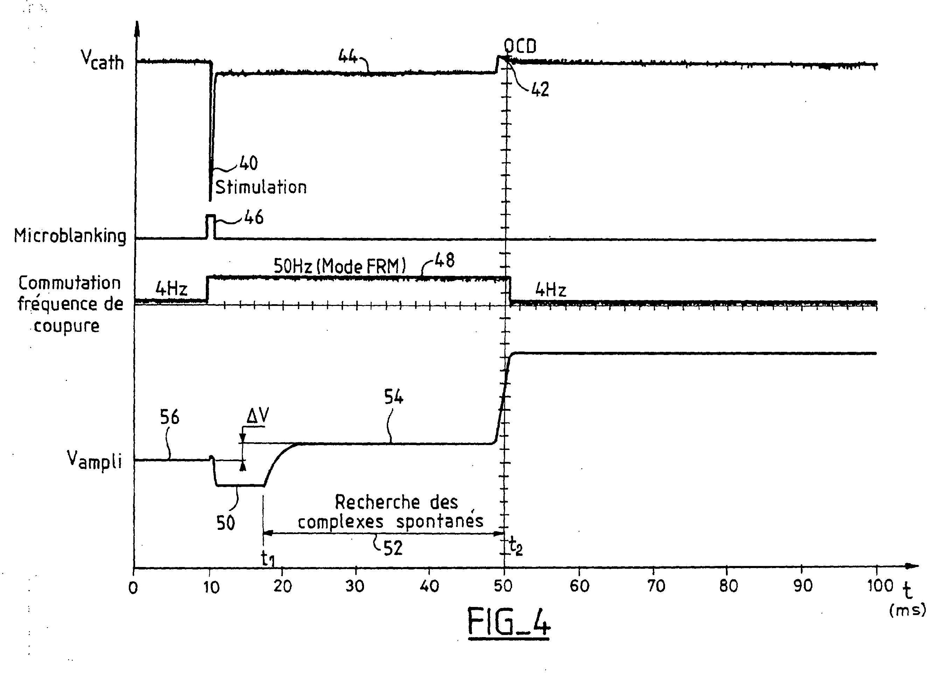

Cette manière de procéder est illustrée notamment figure 3, où l'on peut voir que la période d'écoute 44 est dorénavant située avant la période d'OCD 42 au lieu de se situer après celle-ci.

Dans le cas de la recherche d'une onde P, le décalage de l'OCD est typiquement de 40 ms environ après la stimulation de l'oreillette.

Dans le cas de la recherche d'une onde R, le décalage de l'OCD après stimulation est plus important (typiquement 60 ms environ après la stimulation du ventricule), puisque l'onde R est moins précoce que l'onde P.

Dans tous les cas, le décalage de l'OCD doit être tel que l'OCD ait lieu pendant la période réfractaire absolue post-ventriculaire ou post-auriculaire (PRAPA ou PRAPV), afin de ne pas provoquer de fausse détection de complexes spontanés. Cette dernière contrainte est toutefois aisée à respecter, puisque les valeurs typiques des périodes réfractaires post-stimulation sont de l'ordre de 150 ms.

De plus, pour éviter une saturation trop importante de l'amplificateur au moment de la stimulation, un blanking très court ou "microblanking" est effectué, débutant quelques dizaines de microsecondes avant le début de l'impulsion de stimulation et se terminant quelques dizaines de microsecondes après la fin de cette impulsion. Ce microblanking permet également de protéger le cas échéant l'entrée analogique de l'amplificateur de détection contre toute surcharge en provenance de l'impulsion de stimulation.

De façon préférentielle, le microblanking de protection est réalisé par un composant externe à la puce intégrée du dispositif, ou par un circuit intégré haute tension distinct dont la densité d'intégration peut être moindre.

Par ailleurs, l'amplificateur 30 utilisé pour la détection est avantageusement un amplificateur commutable en un mode rapide de désaturation, dit "FRM" (*Fast Recovery Mode*), préalablement à l'impulsion de stimulation, ce mode étant maintenu pendant la recherche de l'onde évoquée.

Cette commutation en un mode rapide de désaturation permet à l'amplificateur de récupérer plus vite après le microblanking effectué lors de la stimulation, typiquement dans un temps inférieur à 8 ms.

Une telle caractéristique permet d'utiliser un seul et même amplificateur de détection pour trois fonctionnalités différentes de la prothèse cardiaque :
- la transmission de l'ECG endocavitaire, qui doit passer les fréquences basses (à partir de 1 Hz environ) ;
- la détection des signaux cardiaques spontanés inhibant le stimulateur en période d'écoute (à partir de 25 Hz environ) ;
- la détection des ondes évoquées, très tôt après les impulsions de stimulation (à partir de 50 Hz environ).

Le mode FRM peut être obtenu de diverses façons, par exemple en augmentant le courant de polarisation de l'amplificateur, en réinitialisant certains réseaux internes pour annuler l'énergie emmagasinée dans les composants capacitifs et/ou, de façon préférentielle et comme illustré ici, en augmentant la fréquence de coupure à une valeur très élevée, par exemple 50 Hz.

Les chronogrammes de la figure 4 illustrent le séquencement de ces différentes fonctions, en montrant, de haut en bas :
- la tension V_{cath} recueillie sur la borne CATH du dispositif, montrant successivement l'impulsion de stimulation 40, la période d'écoute 44 des ondes évoquées et la période d'OCD 42 ;
- la commande du microblanking par l'impulsion 46 encadrant le pic de stimulation 40 ;
la commutation de la fréquence de coupure de l'amplificateur (4 Hz en mode normal, 50 Hz en mode FRM), le créneau 48 correspondant à la durée pendant laquelle l'amplificateur est commuté en mode FRM) ;
- la tension Vₐₘₚₗᵢ recueillie en sortie de l'amplificateur de détection : après le transitoire 50 inévitable pendant les 8 premières millisecondes, la plage d'écoute 52 comprise entre 8 et 40 ms est ensuite libre de tout post-potentiel de stimulation et permet de détecter sans ambiguïté la présence d'un complexe évoqué.

L'amplificateur est laissé en mode FRM pendant toute la période 44 d'écoute du complexe évoqué ; les signaux évoqués sont alors transmis en sortie de l'amplificateur de détection car ils sont très rapides, et ils seront traités au sein du microcontrôleur 38 par un algorithme spécifique.

Dans ce mode FRM, un ECG sinusal serait transmis très déformé, et une fibrillation atriale serait très atténuée et donc difficile à détecter.

Pour cette raison, l'amplificateur de détection est recommuté dans son mode normal d'écoute pour la détection des ondes spontanées et l'enregistrement de l'ECG, dès que la période de recherche du complexe évoqué est terminée.

C'est à ce moment que l'OCD peut être commandée.

De façon préférentielle, le dispositif quitte le mode FRM de l'amplificateur juste après le début de l'OCD (et non concurremment à ce début), afin de ne pas enchaîner deux types de perturbations. En effet, le retour du mode FRM au mode normal va engendrer une perturbation importante de la sortie de l'amplificateur, et il est important d'être toujours dans une période réfractaire du dispositif. De préférence, on utilise un système à période réfractaire redéclenchable dont la durée est automatiquement ajustée en fonction de la perturbation présente en sortie de l'amplificateur de détection, comme cela est par exemple décrit dans le EP-A-0 962 235 (ELA Médical). Cela étant, dans de nombreux cas une durée de période réfractaire de 150 ms reste suffisante pour des sondes à polarisation moyenne à faible.

De façon avantageuse, on utilise pour la recherche de l'onde évoquée la technique particulière que l'on va maintenant décrire.

Comme on peut le voir sur la trace du bas du chronogramme de la figure 4, le potentiel Vₐₘₚₗᵢ en sortie de l'amplificateur de détection durant la période de recherche 52 de l'onde évoquée comprend un transitoire rapide 50, récupéré en environ 10 ms, suivi d'un niveau de potentiel intermédiaire 54 quasi-statique.

Ce niveau de potentiel intermédiaire 54 est toutefois différent du niveau de potentiel de base 56 au repos. La valeur absolue du potentiel intermédiaire 54 varie en fonction de la nature de la sonde et de sa polarisation, de sorte que la détection de l'onde évoquée ne peut pas se faire simplement en détectant le franchissement d'un seuil par le signal Vₐₘₚₗᵢ en sortie de l'amplificateur.

Le système doit donc être étalonné en évaluant régulièrement le niveau du potentiel intermédiaire 54 au cours de la vie de l'implant.

Une variante avantageuse à cet étalonnage consiste à analyser les échantillons de signal après numérisation pour en déduire un paramètre, par exemple et de préférence, comme illustré sur les figures 5A et 5B, une dérivée seconde évaluée entre deux instants t₁ et t₂ programmables, inclus dans la période 52 de recherche des complexes spontanés. Une dérivée seconde est aisée à calculer à partir d'échantillons numérisés, de sorte que cette technique peut être mise en oeuvre de façon simple par un algorithme approprié au sein du microcontrôleur 38.

Comme on peut le voir en comparant les figure 5A et 5B, l'onde évoquée apparaîtra très nettement à l'examen du signal numérisé dans la zone centrale. Cette onde, si elle est présente, apparaît de manière très distincte en 58.

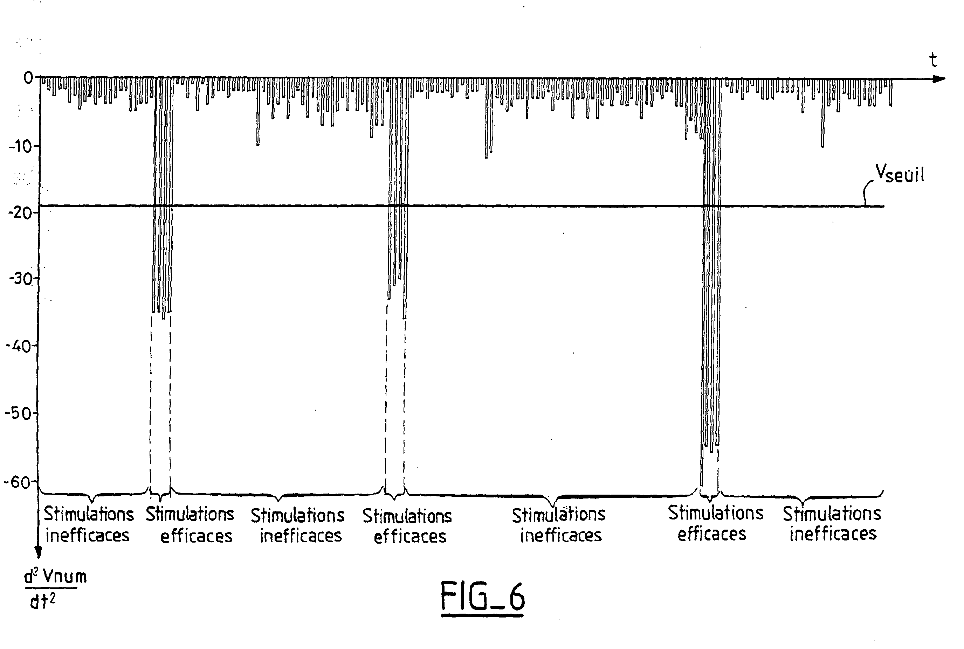

La figure 6 illustre un résultat clinique avec alternance de stimulations inefficaces et efficaces. Les stimulations efficaces (4 stimulations consécutives) donnent des valeurs d'extrema (de minima en l'occurrence) de la dérivée seconde très importants, alors que les simulations non efficaces donnent de faibles valeurs de ces extrema. Le critère de détection de l'onde évoquée, c'est-à-dire de l'efficacité de la stimulation, se traduit alors simplement par le franchissement d'une valeur de seuil Vₛₑᵤᵢₗ de la dérivée seconde. Les cas où la dérivée seconde franchit le seuil correspondent indubitablement au cas où l'onde P évoquée est présente (figure 5B) ; en revanche, lorsque la stimulation est inefficace (figure 5A) la dérivée seconde reste très en dessous du seuil fixé.

## Revendications

1. Un dispositif médical implantable actif, notamment un stimulateur cardiaque, défibrillateur, cardioverteur ou dispositif multisite, comprenant :
- une borne (CATH) apte à être reliée à une électrode intracardiaque d'une sonde implantée dans une première cavité cardiaque ;
- des moyens de stimulation (12-18), aptes à appliquer une impulsion de stimulation à la borne pour délivrance de cette impulsion à la première cavité ;
- des moyens de décharge (24, 26) couplés à la borne, aptes à court-circuiter cette borne pendant la durée d'une période de décharge (42, OCD) ;
- au moins un étage apte à détecter sur la borne des signaux électriques correspondant à des potentiels spontanés apparaissant dans la première cavité, et comportant :
· un amplificateur (30) comportant une entrée couplée à la borne, et une sortie, et
· des moyens de blanking (34), comportant un commutateur apte à découpler temporairement la borne d'avec l'entrée de l'amplificateur ; et
- des moyens séquenceurs, aptes à, successivement :
· activer les moyens de décharge en réponse à l'application de l'impulsion de stimulation (40), pendant la durée de la période de décharge, et
· activer les moyens de blanking pendant au moins la durée de la stimulation et la période de décharge,
dispositif **caractérisé en ce que**, les moyens séquenceurs ayant activé les moyens de blanking une première fois (MICRO-BLANKING) pendant la durée de l'impulsion de stimulation (40), ces moyens séquenceurs sont aptes, en outre, à :
· activer les moyens de décharge avec délai après l'application de l'impulsion de stimulation, puis
· inhiber les moyens de blanking pendant une période d'écoute (44) incluant au moins une partie dudit délai, de manière à autoriser la détection par l'amplificateur, pendant cette période d'écoute, des signaux correspondant à des potentiels spontanés post-stimulation dans la première cavité, et enfin
· activer les moyens de blanking une seconde fois (BLANKING) après la période d'écoute, pendant la durée de la période de décharge (42, OCD).

2. Le dispositif de la revendication 1, dans lequel :
- l'amplificateur (30) est un amplificateur commutable entre un mode de détection normale et un mode de désaturation rapide, et
- les moyens séquenceurs sont aptes, en outre, à
· commuter l'amplificateur en mode de désaturation rapide pendant au moins la durée de la période d'écoute.

3. Le dispositif de la revendication 2, dans lequel le mode de détection normale est un mode à fréquence de coupure basse et le mode de désaturation rapide est un mode à fréquence de coupure élevée.

4. Le dispositif de la revendication 2, dans lequel les moyens séquenceurs sont aptes à recommuter l'amplificateur en mode de détection normale après le début de la période de décharge, de façon non concomitante à ce début.

5. Le dispositif de la revendication 1, dans lequel les moyens séquenceurs commandent les moyens de décharge de manière à mettre fin à la période de décharge avant la fin d'une période réfractaire post-stimulation d'analyse du signal en sortie de l'amplificateur.

6. Le dispositif de la revendication 5, dans lequel les moyens séquenceurs mettent fin à la période de décharge avant la fin de la période réfractaire post-stimulation d'analyse du signal en sortie de l'amplificateur, à la fois pour ladite première cavité et pour au moins une deuxième cavité cardiaque, de manière à éviter les fausses détections dans cette (ces) deuxième(s) cavité(s).

7. Le dispositif de la revendication 5, dans lequel la période réfractaire est une période réfractaire redéclenchable de durée variable en fonction du niveau de perturbation présent en sortie de l'amplificateur.

8. Le dispositif de la revendication 1, comportant des moyens d'analyse du signal en sortie de l'amplificateur pendant la période d'écoute, aptes à détecter la présence d'une onde évoquée consécutive à la stimulation appliquée à la cavité, de manière à discriminer entre stimulations efficaces et stimulations non efficaces.

9. Le dispositif de la revendication 8, dans lequel les moyens d'analyse comportent des moyens aptes à analyser les variations d'une dérivée d'ordre *n*, avec *n* = 1, de préférence *n* = 2, du signal en sortie de l'amplificateur.

10. Le dispositif de la revendication 9, dans lequel les moyens d'analyse comportent en outre des moyens aptes à détecter le franchissement d'un seuil (Vₛₑᵤᵢₗ) par un extremum de ladite dérivée du signal en sortie de l'amplificateur.

## Patentansprüche

1. Aktive implantierbare medizinische Vorrichtung, insbesondere Herzschrittmacher, Defibrillator, Kardioverter oder Multisite-Vorrichtung, mit:
- einem Anschluss (CATH), der mit einer intrakardialen Elektrode einer in einem ersten Herzraum implantierten Sonde verbindbar ist;
- Stimulationsmitteln (12-18), die geeignet sind, an den Anschluss einen Stimulationsimpuls zur Abgabe dieses Impulses an den ersten Raum anzulegen;
- an den Anschluss gekoppelten Entladungsmitteln (24, 26), die geeignet sind, diesen Anschluss während der Dauer einer Entladungsperiode (42, OCD) kurz zu schließen;
- wenigstens einer Stufe, die geeignet ist, am Anschluss elektrische Signale zu erfassen, die spontanen Potentialen entsprechen, welche im ersten Raum auftreten, und folgendes umfasst:
■ einen Verstärker (30) mit einem an den Anschluss gekappelten Eingang und einem Ausgang, und
■ Austastmittel (34), mit einem Taster, der geeignet ist, den Anschluss zeitweise vom Eingang des Verstärkers zu entkoppeln; und
- Sequenziermitteln, die geeignet sind, nacheinander:
■ die Entladungsmittel in Antwort an das Anlegen des Stimulationsimpulses (40) während der Dauer der Entladungsperiode zu aktivieren, und
■ die Austastmittel während wenigstens der Dauer der Stimulation und der Entladungsperiode zu aktivieren,
Vorrichtung, die **dadurch gekennzeichnet ist, dass**, wenn die Sequenziermittel die Austastmittel ein erstes Mal (Mikro-Austasten) während der Dauer des Stimulationsimpulses (40) aktiviert haben, die Sequenziermittel außerdem geeignet sind:
■ die Entladungsmittel mit Verzögerung nach Anlegen des Stimulationsimpulses zu aktivieren, sodann
■ die Austastmittel während einer Abhörperiode (44) zu hemmen, die wenigstens einen Teil der Verzögerung umfasst, um so während dieser Abhörperiode die Erfassung von Signalen durch den Verstärker zu erfauben, die spontanen Potentialen nach der Stimulation im ersten Raum entsprechen, und schließlich
■ die Austastmittel ein zweites Mal (Austasten) nach der Abhörperiode während der Dauer der Entladungsperiode (42, OCD) zu aktivieren.

2. Vorrichtung nach Anspruch 1, in welcher:
- der Verstärker (30) ein Verstärker ist, der zwischen einem normalen Erfassungsmodus und einem Modus schneller Entsättigung umschaltbar ist, und
- die Sequenziermittel außerdem geeignet sind, den Verstärker wenigstens während der Dauer der Abhörperiode in den Modus schneller Entsättigung umzuschalten.

3. Vorrichtung nach Anspruch 2, in welcher der normale Erfassungsmodus ein Modus mit einer niedrigen Grenzfrequenz ist und der Modus schneller Entsättigung ein Modus mit einer hohen Grenzfrequenz ist.

4. Vorrichtung nach Anspruch 2, in welcher die Sequenziermittel geeignet sind, den Verstärker nach dem Beginn der Entladungsperiode in den normalen Erfassungsmodus zurückzuschalten, auf zu diesem Beginn nicht gleichzeitige Weise.

5. Vorrichtung nach Anspruch 1, in welcher die Sequenziermittel die Entladungsmittel befehligen, um so die Entladungsperiode vor dem Ende einer unempfänglichen, post-stimulativen Periode zur Analyse des Signals am Ausgang des Verstärkers zu beenden.

6. Vorrichtung nach Anspruch 5, in welcher die Sequenziermittel die Entladungsperiode vor dem Ende einer unempfänglichen, post-stimulativen Periode zur Analyse des Signals am Ausgang des Verstärkers beenden, sowohl für den ersten Raum wie auch für wenigstens einen zweiten Herzraum, um so Falscherkennungen in diesem (diesen) zweiten Raum(Räumen) zu vermeiden.

7. Vorrichtung nach Anspruch 5, in welcher die unempfängliche Periode eine wieder auslösbare unempfängliche Periode ist, von in Abhängigkeit des am Ausgang des Verstärkers vorhandenen Störungsniveaus veränderlicher Dauer.

8. Vorrichtung nach Anspruch 1, mit Mitteln zur Analyse des Signals am Ausgang des Verstärkers während der Abhörperiode, die geeignet sind, das Vorliegen einer im Anschluss an die an den Raum angelegte Stimulation gebildeten Welle zu erfassen, um so zwischen wirksamen Stimulationen und unwirksamen Stimulationen zu unterscheiden.

9. Vorrichtung nach Anspruch 8, in welcher die Analysemittel Mittel umfassen, die geeignet sind, die Veränderungen einer Ableitung der Ordnung *n*, mit *n* = 1, vorzugsweise *n* = 2, des Signals am Ausgang des Verstärkers zu analysieren.

10. Vorrichtung nach Anspruch 9, in welcher die Analysemittel außerdem Mittel umfassen, die geeignet sind, die Durchschreitung eines Schwellenwerts (V_{schwelle}) durch einen Extremwert der Ableitung des Signals am Ausgang des Verstärkers zu erfassen.

## Claims

1. An active implantable medical device, in particular a cardiac pacemaker, defibrillator, cardioverter or multisite device, comprising:
- a terminal (CATH) connectable to an intracardiac electrode of a probe, implanted in a first cardiac cavity;
- stimulation means (12-18) able to apply a stimulation pulse to said terminal for delivery of said pulse to said first cavity;
- discharging means (24, 26) coupled to said terminal being able to short-circuit said terminal for the duration of a discharge period (42, OCD);
- at least one stage, being able to detect electric signals at said terminal corresponding to spontaneous potentials appearing in said first cavity, and including:
■ an amplifier (30) comprising an input coupled to said terminal and an output, and
■ blanking means (34) comprising a switch able to temporarily uncouple the terminal from the amplifier input; and
- sequencer means able to, successively:
■ activate the discharging means in response to an application of the stimulation pulse (40), throughout the duration of the discharge period, and
■ activate the blanking means throughout at least the duration of the stimulation and the discharging period,
device, **characterised in that**, the sequencer means having activated the blanking means for a first time (MICRO-BLANKING) throughout the duration of the stimulation pulse (40), said sequencer means is furthermore able:
■ to activate the discharging means with a delay after the application of the stimulation pulse, then
■ to inhibit the blanking means throughout a listening period (44), comprising at least a part of said delay, in order to allow, during this period of listening, the detection of the signals corresponding to spontaneous post-stimulation potentials in the first cavity by the amplifier, and finally
■ to activate the blanking means a second time (BLANKING) after the listening period, throughout the duration of said discharge period (42, OCD).

2. The device of claim 1, wherein:
- said amplifier (30) is an amplifier commutable between a normal detection mode and a fast desaturation mode, and
- the sequencer means further is able to commute the amplifier into said fast desaturation mode throughout at least the duration of the listening period.

3. The device of claim 2, wherein the normal detection mode is a mode with a low cut-off frequency and the fast desaturation mode is a mode with a high cut-off frequency.

4. The device of claim 2, wherein the sequencer means is able to commute the amplifier back into said normal detection mode after the beginning of the discharge period, in a way that is non-concomitant to said beginning.

5. The device of claim 1, wherein the sequencer means commands the discharging means in such a way as to terminate the discharge period before the end of a post-stimulation refractory period of analysis of the amplifier output signal.

6. The device of claim 5, wherein said sequencer means terminates the discharge period before the end of the post-stimulation refractory period of analysis of the amplifier output signal, at the same time for the first cavity and for at least a second cardiac cavity, so as to avoid false detections in said second cavity(ies).

7. The device of claim 5, wherein said refractory period is a re-triggerable refractory period having a variable duration according to the level of disturbance present at amplifier output.

8. The device of claim 1, comprising means for analyzing the amplifier output signal during the listening period, being able to detect the presence of an evoked wave consecutive to the stimulation applied to said cavity, so as to discriminate between effective stimulations and non-effective stimulations.

9. The device of claim 8, wherein the means for analyzing comprises means able to analyze the variations of a derivative of order *n*, with *n* = 1, preferably *n* = 2, of the amplifier output signal,

10. The device of claim 9, wherein the analyzing means further comprises means able to detect the crossing of a threshold (V_{threshold}) by an extremum of the aforementioned derivative of the amplifier output signal.
